# EUROPEAN PATENT SPECIFICATION

(11) **EP 4 469 015 B1**
(45) Date of publication and mention of the grant of the patent: **21.01.2026**
(21) Application number: 23701928.6
(22) Date of filing: 24.01.2023
(51) Int. Cl.: A61K 8/04, A61K 8/97, A61Q 17/04

(54) **COMPOSITION CONTAINING NERO DI TROIA POMACE EXTRACT**
ZUSAMMENSETZUNG MIT EINEM EXTRAKT AUS DER NERODI TROIA TRESTERA
COMPOSITION CONTENANT UN EXTRAIT DE MARC DE NERO DI TROIA

(30) Priority: 25.01.2022 IT 202200001190
(43) Date of publication of application: 04.12.2024
(73) Proprietor: Planbio Cosmetics Srl, 70123 Bari (BA) (IT)
(72) Inventor: BOTTALICO, Michele, 70123 Bari (BA) (IT); CELLAMARE, Saverio, 70123 Bari (BA) (IT)
(74) Representative: Tagliafico, Giulia
(86) International application number: PCT/EP2023/051627
(87) International publication number: WO 2023/144120

(56) References cited:
- FR-A1- 3 096 582
- EYIZ VILDAN ET AL: "Optimization of green extraction of phytochemicals from red grape pomace by homogenizer assisted extraction", JOURNAL OF FOOD MEASUREMENT AND CHARACTERIZATION, SPRINGER US, BOSTON, vol. 14, no. 1, 10 September 2019 (2019-09-10), pages 39 - 47, XP037025768, ISSN: 2193-4126, [retrieved on 20190910], DOI: 10.1007/S11694-019-00265-7

## Description

### TECHNICAL FIELD OF THE INVENVION

The present invention relates to a gel composition for topical use comprising active ingredients for the prevention of damage from oxidative stress due to sun exposure and for the prevention of skin tumours. The invention relates in particular to a composition containing a glyceric extract of *Vitis vinifera* obtained from the pomace of the Nero di Troia vine and xanthan gum, optionally with the addition of sunscreens, in which the glyceric extract is optionally obtained by ultrasound extraction.

### BACKGROUND OF THE INVENTION

It is known that inflammatory factors and oxidative stress, also stimulated by exposure to sunlight, are involved in the formation and proliferation of skin neoplasms. The antioxidative effect of resveratrol is also known.

Patent application US2016287531 A1 reports methods of treating skin disorders by administering a composition, in particular in the form of a nano-emulsion, comprising a therapeutic amount of a substituted cis- or trans-stilbene or a hybrid stilbene, preferably an analogue of resveratrol, and at least one antibiotic. The claimed compounds and methods are indicated to be useful for treating skin conditions characterized by inflammation, particularly tumours. Previous studies on resveratrol, in the context of carcinogenesis and as a chemo-preventive agent, have shown that this molecule inhibits the three stages of carcinogenesis. Resveratrol has shown anti-oxidative properties, to which the anti-inflammatory activity is also attributed.

Patent application US2019201318 A1 mentions a cosmetic composition for topical use to counteract the signs of aging, containing, in addition to other active ingredients present in the fir extract, polyphenols selected from monomers and oligomers of proanthocyanidins, hydroxystilbenes, flavonoid monomers and oligomers and their derivatives and mixtures.

Patent application US2010310615 A1 relates to compositions for cosmetic, dietetic and therapeutic use comprising polyphenolic stilbenoid derivatives, useful for preventing and controlling pathologies and aging of living organisms and tissues. Patent application FR 3096582 A1 discloses compositions for skin care comprising an extract from vitus vinifera grape pomace from Chateau d'Yquem.

The use in the pharmaceutical, nutraceutical and cosmetic fields of polyphenols and in particular resveratrol from *Vitis Vinifera* extracts is known.

The stilbene polyphenols concentration in these extracts varies according to the grapevine variety and also according to the parts of the plant, from which the extract is obtained.

A comparative study on the flavonoids and stilbene content of the red grape varieties Susumaniello, Uvalino and Nero di Troia (specific small acinus biotype), conducted by Suriano et al. [Conference Paper, August 2011, www.researchgate.net/publication/271518705] on different parts of the acinus (peel, seeds and juice) showed that nutraceutical substances present in the three varieties show significant differences in terms of quality and quantity. In particular, Uvalino is characterized by the higher content of stilbene compounds, while the other two varieties have a higher average concentration than other Italian red grape varieties and Nero di Troia has the highest content of total flavonoids, polyphenols and proanthocyanidins.

Studies conducted by Antonacci et al. (L'Enologo, n.°3 - March 2015) show that the variety of *Vitis Vinifera* Nero di Troia has a high polyphenolic concentration, even higher in the small acinus variety than in the large acinus variety.

The different extraction methods also have an impact on the concentrations of recovered polyphenols, especially resveratrol, as shown by Roselli et. al (Clinical Immunology, Endocrine & Metabolic Drugs, 2015, 2, 8-12) in a comparative study between microwave-assisted extraction and extraction by convection heating, applied to the pomace of Uva di Troia Canosina.

Catalan et. al (Hindawi Publishing Corporation, ISRN Analytical Chemistry, Volume 2013) conducted a study to evaluate the polyphenolic content of "Uva Di Troia Canosina". The seeds and peels, collected at four different stages of the fermentation process, were extracted by maceration, and the purified extracts were analysed to study the influence of fermentation on the polyphenol content. Seed extraction was performed by multistep maceration with ethanol and acetone, furthermore the extracts were purified with pure ethyl acetate. The extraction of the peels, on the other hand, was obtained by maceration in a single step in methanol and purification with a brominated synthetic adsorbent resin. The evaluation of the extraction yield and the polyphenolic content was carried out by TLC (ThinLayerChromatography), UV/VIS (UltraViolet/VISible) and LC/DAD (Liquid Chromatography with photoDiode Array Detection) analysis. The characteristic polyphenols (catechin, epicatechin and procyanidin B1 and B2) were present in the seed extracts, useful for the development of a nutraceutical product, endowed with antioxidant properties, while no resveratrol was found in the Troia canosine grape skin extracts, also in an LC/MS-MS analysis (Liquid Chromatography - tandem Mass Spectrometry).

### DESCRIPTION OF THE INVENTION

Object of the present invention is a gel composition for topical use comprising xanthan gum, a glyceric extract of pomace of *Vitis vinifera* of the Nero di Troia vine together with pharmaceutically acceptable excipients.

According to one aspect of the invention, the glyceric extract is present in the composition in an amount ranging from 10% to 60% by weight, preferably from 20% to 50% by weight.

According to another aspect in the composition xanthan gum is present in an amount from 0.5% to 4% by weight, preferably from 1% to 2% by weight.

Moreover, according to a further aspect, the composition of the invention comprises UVA, UVB sunscreens in quantities sufficient to obtain SPF 50 protection.

A further object of the present invention is the composition described above for use in the prevention of damage from oxidative stress due to sun exposure and for the prevention of skin tumours. Skin cancer is the most common type of cancer and generally develops in areas of skin exposed to the sun and people with fair skin (less skin pigmentation) are more at risk. The most frequent forms of skin cancer are basal cell carcinoma (about 80%), squamous cell carcinoma (about 16%) and melanoma (about 4%).

The glyceric extract of pomace of *Vitis vinifera* of the Nero di Troia vine is preferably obtainable by ultrasound extraction at room temperature for 4-10 minutes in a mixture of 50-80% by weight of glycerine and 20-50% by weight of water.

The process for the preparation of said glyceric extract of the pomace of *Vitis vinifera* of the Nero di Troia vine includes, in fact, extraction with ultrasound at room temperature for 4-10 minutes in a mixture of 50-80% by weight of glycerin and 20-50 % by weight of water.

A further object of the present invention is the glyceric extract obtained through this method.

The process for the preparation of the composition of the present invention comprises the mixing of xanthan gum with the glyceric extract of pomace of *Vitis vinifera* of the Nero di Troia vine together with pharmaceutically acceptable excipients.

The term "pomace" here refers to the residue from the pressing of the grapes consisting of stalks, skins, seeds also containing a certain quantity of wine or fermented must.

Therefore, the present invention also has the advantage of using natural and waste raw materials, which derive from the preparation of wine. The pomace is in fact used to prepare the glycolic extract of the present invention without the need to separate the stalks, from the peels or from the seeds.

The term "xanthan gum" or xanthan is here meant to refer to a complex polysaccharide consisting of hexoses, mainly D-glucose and D-mannose, and D-glucuronic and pyruvic acids. This high molecular weight polysaccharide is obtained by bacterial fermentation of a simple carbon hydrate, such as glucose or sucrose. Xanthan gum is also used in the food sector, where it is used as a thickening and stabilizing additive, marked by the code E415. A minimal addition of this additive (0.5-1% or less) will greatly increase the viscosity of a liquid composition. Xanthan gum is commercially available from various suppliers, such as Bema Cosmetici srl based in Borsea-Rovigo.

The term "pharmaceutically acceptable excipients" is here meant to refer to excipients of the conventional type, i.e. compounds inert towards the active ingredient and the pharmaceutical form and non-toxic for the subject to whom they are administered. In the case of the present invention useful classes of such excipients are for example: diluents (compounds added when the mass of the active ingredient is not sufficient for the preparation of the composition) and antioxidants-antimicrobials (used to extend the shelf life of the product). Examples of such pharmaceutically acceptable excipients are given in Remington's Pharmaceutical Sciences, 18th Ed. (1990, Mack Publishing Co., Easton, Pa. 18042, pages 1435-712).

According to the data generated by the inventors and herein reported in the section "Examples", the glyceric extract of pomace of *Vitis vinifera* of the Nero di Troia vine led to a statistically significant reduction in the levels of reactive oxygen species (ROS) in an *in vitro* model of antioxidant activity on human keratinocytes.

Moreover, a study carried out by the inventors and herein reported in the section "Examples" quantifies substances, such as eicosapentaenamide (EPM), 24-hydroxyarachidonate (24-HAC) and ginkgolic acid (GKA), present in the glyceric extract of pomace of *Vitis vinifera* of the Nero di Troia vine. In particular, the presence of EPM and 24-HAC, derivatives of polyunsaturated acids, and GKA (a 6-tridecylsalicylic acid), which can be used as surfactant-cleansing and emulsifying agent in cosmetics led the inventors to prepare the composition of the invention, i.e. a gel composition for topical use comprising xanthan gum, a glyceric extract of pomace of *Vitis vinifera* of the Nero di Troia (NdT) vine together with pharmaceutically acceptable excipients.

Finally, the finding that pomace extracts, and in particular that of NdT, can be used as new and promising natural sources of polyunsaturated acid derivatives to modulate rheological properties in cosmetic production renders this innovation a virtuous example of circular green economy.

Some (non-limiting) examples of the present invention are provided below.

### DESCRIPTION OF THE DRAWINGS

Figure 1 - Figure 1 reports the 3D pie charts representing the content of flavonoids and terpenoids of glyceric extracts obtained as described in Example 1 from pomaces of Nero di Troia (NdT) (1a) and Primitivo (1b).
Figure 2 - Figure 2 reports the 3D pie charts representing the content of flavonoids and terpenoids of glyceric extracts obtained as described in Example 1 from pomaces of Aglianico (2a) and Falanghina (2b).
Figure 3 - Figure 3 reports the ESI (Electrospray ionization) mass spectrum (positive ion scan) of the NdT pomace extract highlighting highest m/z signals (with a cutoff at 10% abundance).
Figure 4 - Figure 4 reports the ESI mass spectrum (positive ion scan) of the Primitivo pomace extract highlighting highest m/z signals (with a cutoff at 10% abundance).
Figure 5 - Figure 5 reports the ESI mass spectrum (positive ion scan) of the Aglianico pomace extract highlighting highest m/z signals (with a cutoff at 10% abundance).
Figure 6 - Figure 6 reports the ESI mass spectrum (positive ion scan) of the Falanghina pomace extract highlighting highest m/z signals (with a cutoff at 10% abundance).

### EXAMPLES

### Example 1 - Preparation of the glyceric extract of the pomace of Vitis vinifera from the Nero di Troia vine

The glyceric extract of pomace of *Vitis vinifera* of the Nero di Troia vine was obtained by preparing a mixture containing 60% by weight of glycerin, 25% by weight of pomace and 15% water.

To this mixture, in order to obtain the extract, ultrasounds were subsequently applied at room temperature for 5 minutes. Subsequently the extract was filtered, obtaining a clear or slightly opalescent liquid with a dark purplish colour.

The following preservatives were then added to said extract: 0.60% by weight of benzyl alcohol, 0.18% by weight of sodium benzoate and 0.12% by weight of potassium sorbate.

The glyeric extract thus obtained (also named as **GExtract NdT)** was used for the assays reported in the following Examples 4-6.

### Example 2 - Preparation of the composition in gel for topical use containing the glyceric extract of pomace of Vitis vinifera of the Nero di Troia vine

The gel for topical use is obtained by diluting the GExtract NdT obtained in Example 1 with water in different proportions depending on the concentration of the compositions to be prepared 1:1, 1:2 or 1:4.

Subsequently to the dilution, xanthan gum is added, little by little, between 1 gram and 2 grams for every 100 grams of liquid mixture prepared and is mixed until the desired density is obtained. Subsequently, the gel obtained is left to rest for 24-48 hours.

### Example 3 - Prevention of oxidative stress damage due to sun exposure

The efficacy of the composition obtained in Example 2 alone or in comparison with other compositions containing other *Vitis vinifera* pomace extracts is evaluated by carrying out one or more of the following tests/assays.

| **Assay** | **Type** | **Measured property** | **Note/Reference** |
|---|---|---|---|
| ABTS^{•}+ scavenging | Chemical-physical | Nonspecific total antioxidant activity | Spectophotometric test based on the interaction between antioxidant species and radical cation ABTS^{•+} generated from potassium persulfate (PP) and ABTS *[2,20-azino-bis (3-ethylbenzothiazoline-6-sulfonic acid)]).* |
| | | | Refs. a) Re, R. et al. Antioxidant activity applying an improved ABTS radical cation decolorization assay. Free Radic. Biol. Med. 1999, 26, 1231-1237; b) Int. J. Mol. Sci. 2020, 21, 1131. |
| DPPH scavenging | Chemical-physical | Nonspecific total antioxidant activity | Spectrophotometric test based on the interaction between antioxidant species and DPPH [2,2-diphenyl-1-(2,4,6-trinitrophenyl)hydrazyl]. |
| | | | Ref. Sagar B. Kedare et al. Genesis and development of DPPH method of antioxidant assay. J Food Sci. Technol. 2011 Aug; 48(4): 412-422. |
| FRAP assay | Chemical-physical | Nonspecific total antioxidant activity | Colorimetric test which measures the antioxidant potential by reducing Fe(III) to Fe(II). |
| | | | Ref. Benzie et al. The ferric reducing ability of plasma (FRAP) as a measure of "antioxidant power": the FRAP assay. Anal. Biochem. 1996 Jul 15;239(1):70-6. |
| ORAC assay | Chemical-physical | Nonspecific total antioxidant activity | Spectrophotometri test (**o**xygen **r**adical **a**bsorbance **c**apacity assay) based on the interaction between antioxidant and peroxide radical scavenger in the presence of a fluorescent indicator). |
| | | | Ref. Ou, B. et al. Development and validation of an improved oxygen radical absorbance capacity assay using fluorescein as the fluorescent probe. Journal of Agricultural and Food Chemistry, 49, 4619-4626 |
| MTT assay | *In vitro* (cell) | Neuroprotective activity vs Hypo E22 cell line (rat hypothalamus) | Spectrophotometric test associated with the cellular viability after exposure to oxidative stress from H₂O₂. |
| | | | Ref. Chiavaroli A. et al. Phenolic Characterization and Neuropro tective Properties of Grape Pomace Extracts. Molecules 2021, 26, 6216. |
| CAA cellular antioxidant assay | *In vitro* (cell) | Antioxidant activity in cancer cell line | Spectrophotometric activity that measures the capacity to prevent the formation of dichlorofluorescein from |
| | | | 2,2'-Azobis(2-amidinopropane) dihydrochloride (ABAP) generated from radicals in human hepatocellular carcinoma cells (HepG2). |
| | | | Ref. Kelly L Wolfe et al. Cellular antioxidant activity (CAA) assay for assessing antioxidants, foods, and dietary supplements. J Agric Food Chem. 2007 Oct 3 1;55(22):8896-907. |
| Nitric oxide assay | *In vitro* (cell) | Antioxidant activity in cancer cell line | Colorimetric test which measures the capacity to prevent the release of NO in rat macrophage cells (RAW 264.7) stimulated by lipopolysaccharides. |
| | | | Ref. Nathan S. Bryan et al. Methods to Detect Nitric Oxide and its Metabolites in Biological Samples. Free Radic Biol Med. 2007 Sep 1; 43(5): 645-657. |
| Screening agents for melanoma prevention | *In vitro* (cell) | Proliferative activity vs melanoma cancer cell lines | Cancer biomarkers reduction (annexin V, human leukocyte antigen-DR; E-cadherin, N-cadherin) in melanocytes subjected to physical (UV) and chemical oxidative stress . |
| | | | Refs. a) E. Elmore et al. Development and characteristics of a human cell assay for screening agents for melanoma prevention. Melanoma Research 2007, 17:42-50. b) G. K. Couto. The Melding of Drug Screening Platforms for Melanoma. Front Oncol. 2019; 9: 512. |

### Example 4 - Effect GExtract NdT on the cellular viability: in vitro study with the HaCaT cells

The aim of this study was to evaluate *in vitro* the effect of GExtract NdT (obtained in Example 1) on the viability of the human keratinocyte cell line HaCaT. The evaluation was performed on cells exposed to GExtract NdT at a dilution of 2% in complete medium for 24 hours.

The cellular viability was measured by MTS colorimetric essay through the absorbance measurement obtained from the conversion of a bioreduced tertazolium compound from viable cells into a coloured formazan soluble in the culture medium.

### Results

The HaCaT cells were exposed for 24 hours to 2% of GExtract NdT of and Triton-X 100 and subsequently the viability was evaluated by MTS assay. The results are reported in Table 1. In the HaCaT cells exposed for 24 hours at increasing concentrations of Triton-X 100 (used as positive control) a marked negative effect on cell viability was recorded already at the lowest tested concentration. The surfactant lysed all the cells starting from the concentration of 0.01%. The treatment with GExtract NdT had no effect on the cell viability at the concentration of 2%.

**Table 1 - GExtract NdT and Triton-X 100 effect on the HaCat cells. The results are expressed as percentage normalized on the control group.**

| Test Item | % (v/v) | % CTR | %STD |
|---|---|---|---|
| | CTR | 100 | 3.3 |
| GExtract NdT | 2 | 95.26 | 4.52 |
| TRITON-X 100 | 0.001 | 84.54 | - |
| | 0.001 | 67.19 | - |
| | 0.005 | 52.098 | - |
| | 0.01 | 0 | - |
| | 0.05 | 0 | - |
| | 0.5 | 0 | - |

### Conclusions

In the study no bacterial contaminations were found in the treatment chamber, while a concentration-dependent reduction in the number of viable cells was recorded in cells treated with the surfactant Triton-X (used here as a cell lysing treatment). The study can therefore be considered valid.

No statistically significant effects were observed in the cells treated with GExtract NdT for 24 hours at 2% v/v concentration.

From the data obtained using the in vitro test described herein and under those experimental conditions, we can conclude that: GExtract NdT did not lead to a statistically significant viability reduction in HaCaT cells exposed for 24 hours to a concentration of 2% v/v.

Example 5 - Antioxidant efficacy of GExtract NdT in an *in vitro* study with the HaCat cells The aim of this study was to evaluate in vitro the antioxidant effect of GExtract NdT (obtained in Example 1) on of the human keratinocyte cell line HaCaT. The evaluation was carried out on cells exposed to a 2% dilution of the formulation for 24 hours and subsequently treated with menadione (as an oxidizing stimulus).

The antioxidant activity was quantified as the reduction of Reactive Oxygen Species (ROS) induced by exposure to menadione alone, by dichlorofluorescein diacetate assay.

### Results

Pre-treating the cells with GExtract NdT at 2% v/v concentration resulted to be protective against both the stimuli: in fact, a reduction of about 20% and of about 5%, respectively, was registered with regard to menadione at 50 µl and 100 µl. The results of the exposure to menadione after pre-treatment with GExtract NdT are reported in Table 2

**Table 2 - Antioxidant effect of GExtract NdT at the concentration of 2% v/v. The results are expressed as relative units of fluorescence.**

| **Menadione (µM)** | **Without GExtract NdT** | | **With GExtract NdT** | | | |
|---|---|---|---|---|---|---|
| | **Average** | **Standard Dev.** | **Average** | **Standard Dev.** | **% Reduction** | **Statistical significance (p value)** |
| 50 | 3131.00 | 50.91 | 2755.67 | 134.84 | 11.99 | < 0.001 |
| 100 | 3430.00 | 46.67 | 3240.20 | 51.29 | 5,53 | < 0.001 |

### Conclusions

The study can be considered valid, because no bacterial contamination was found in the treatment chamber and an increased fluorescence, concentration-dependent, was recorded in the cells treated with menadione (here used as an oxidative treatment).

With regard to antioxidant efficacy, a positive effect was found in cells pre-treated with GExtract NdT. Said effect was marked in pre-treatment with GExtract NdT at 2% v/v, with a percentage reduction of oxidative stress of about 12 and 5%, respectively, against menadione at 50 to 100 µM.

From the results obtained using the *in vitro* described herein and in those experimental conditions, we can conclude that: GExtract NdT led to a statistically significant reduction in levels of reactive oxygen species (ROS) after 24 hours from treatment with the non-cytotoxic concentration tested in menadione-stimulated human keratinocytes.

In the light of what emerged in our experimental conditions, the antioxidant activity of GExtract NdT is confirmed.

### Example 6: - Comparative study on the flavonoids and terpenoids content of four grapevine varieties extracts

The glyceric extracts obtained from four different varieties of vines (Nero di Troia [NdT], Primitivo, Aglianico and Falanghina) were prepared using the same protocol as described in Example 1, and stored at -20°C until the start of the analysis cycle. An exactly weighed amount of each extract was analysed through MS-ESI-QTOF spectroscopy to define the relative profiles of the most representative substances in these matrices, such as polyphenols, terpenes and the main metabolites.

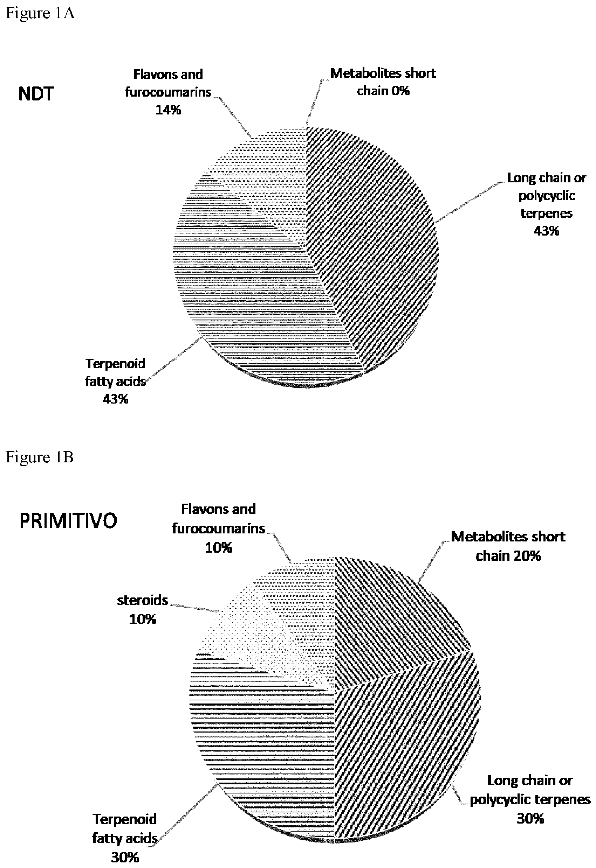

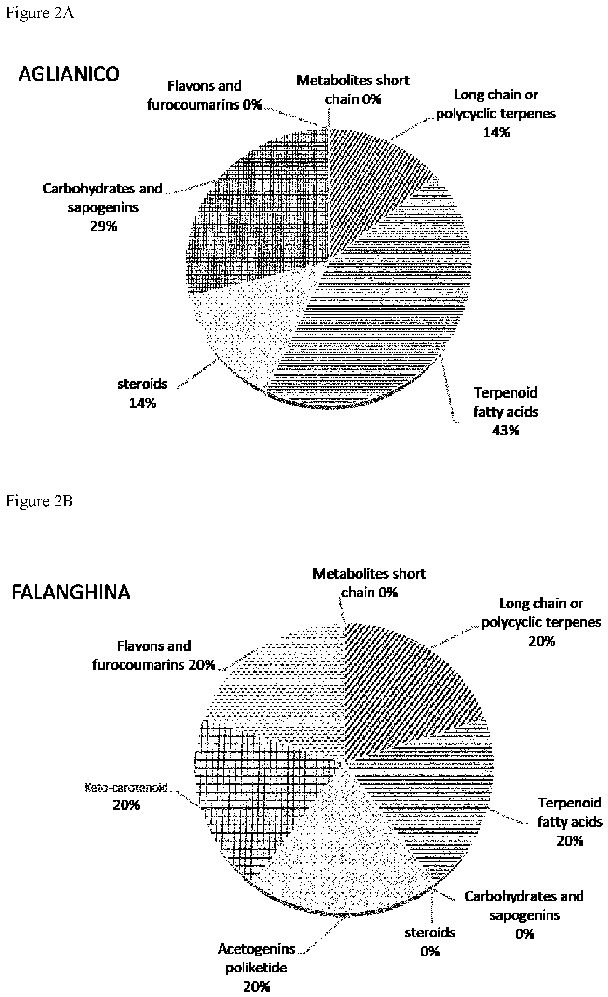

The results are also reported in the following Tables A to D and in the 3D pie charts reported in Figures 1 and 2. In particular, the following tables report the name of some of the compounds tentatively identified by the exact monoisotopic mass formula (ChemCalc).

**TABLE A**

| **Pomace** | **Formula** | **Exp m/z [M+H]⁺** | **Calc m/z [M+H]⁺** | **Example of a compound of natural origin** (class) | **References** |
|---|---|---|---|---|---|
| | C₂₀H₃₁O₃ | **320.237** | **320.235** | 20-hydroxyarachidonate (*fatty acid*) | PubChem CID 40490645 |
| | C₂₂H₃₄O₃ | **321.237** | **321,242** | Isocupressic acid o( Ginkgolic Acid (C13:0) (*terpenoid acid*) | D. Tsimogiannis and V. Oreopoulou Polyphenols in Plants. 2019 Elsevier |
| | C₂₀H₃₆O₅ | **357.259** | **357.2641** | Eunicellanetetrol (*tricyclic terpene*) | Dictionary of Natural Products Supplement 1-1995 |
| **NdT** | C₂₂H₃₂O₄ | **361.241** | **361,2408** | Macrophorin A (*terpene cyclohexenone epox*) | T. Sassa et al. Agric. Biol. Chem.,47(I), 187~189, 1983 |
| | C₁₉H₃₆O₇ | **377.2582** | **377.2539** | Myrsinionoside D (*cyclohexylbutan glycoside*) | PubChemCID10384912 |
| | C₂₃H₄₀O₄ | **381.290** | **381.2998** | Persin (fatty acid) | PubChemCID5283266 |
| | C₂₂H₃₃O₆ | **393.241** | **393.2277** | Mangromicin E4 (*cyclopentadecane skeleton tetrahydrofuran unit*) | T. Nakashima et al. The Journal of Antibiotics (2014) 67, 533-539 |
| | C₂₉H₂₄O₅ | **453.1676** | **453.1701** | Chamuvaritin (*flavanone*) | PubChem CID100418 |

**TABLE B**

| **Pomace** | **Formula** | **Exp m/z [M+H]⁺** | **Calc m/z [M+H]⁺** | **Example of a compound of natural origin** (class) | **References** |
|---|---|---|---|---|---|
| | C₈H₁₀O₆ | **203.0542** | **203,0555** | Succinyl-acetoacetate | Metabolism Vitis vinifera (wine grape).KEGG-T01084: 100244395 |
| | C₁₁H₁₈O₇ | **263.1120** | **263,1130** | Succinyl-D-diginose | K. Ishii et al. The Journal Of Antibiotics Apr. 1983 |
| | C₂₂H₃₄O₃ | **321.237** | **321,2429** | Isocupressic acid o( Ginkgolic Acid (C13:0) (*terpenoid acid*) | D. Tsimogianniset al Polyphenols in Plants. 2019 Elsevier |
| **Primitivo** | C₁₇H₁₇O₁₀ | **381.082** | **381.0801** | 5-O-b-D-glucopyranosyl-8 hydroxypsoralen | Boeira et al. Ciência Rural, v.52, n.9, 2022 |
| | C₂₃H₄₀O₄ | **381.3007** | **381.2998** | Persin (fatty acid) | PubChemCID5283266 |
| | C₂₆H₃₆O₃ | **397.274** | **397.2742** | Strongylophorine-24 (meroterpenoids) | M. Birringer et al. RSC Adv., 2018, 8, 4803 |
| | C₂₇H₄₈O₅ | **453.3723** | **452.3501** | Chimerol or Bufol (cholestane analogs) | Dictionary of Marine Natural Products |
| | C₃₅H₂₆O₁₀ | **607.1600** | **607.1603** | 6"-(2-hydroxy-3-methyl-3-butenyl)-amentoflavone | Al-Shagdari et al. Natural Product Communications Vol. 8 (9) 2013 |

**TABLE C**

| **Pomace** | **Formula** | **Exp m/z [M]⁺/[M+H]⁺** | **Calc m/z [M]⁺/[M+H]⁺** | **Example of a compound of natural origin** (class) | **references** |
|---|---|---|---|---|---|
| **Aglianico** | C₁₇H₃₄O₅ | **318.222** | **318.2406** | Aleuritic acid methyl ester (*fatty acid*) | G. Tedeschi et al. ACS Sustainable Chem. Eng. 2018, 6, 11, 14955-14966 |
| | | | **318.2042** | Seprilose (*carbohydrate*) | SEPRILOSE(ncats.io) |
| | C₂₀H₃₁O₃ | **320.234** | **320.235** | 20-hydroxyarachidonate (*fatty acid*) | PubChem CID40490645 |
| | C₂₀H₃₂O₃ | **321.240** | **321.242** | Isocupressic acid o( Ginkgolic Acid (C13:0) *terpenoid acid*) | R. Nicoletti et al. Agriculture 2015, 5(4), 918-970; |
| | C₂₂H₄₀O₆ | **401.288** | **401.2902** | Chalmicrin (*mannitol ether of monocyclofarnesol*) | T. Fex, Phytochemistry, Volume 21, Issue 2, 1982, 367-369 |
| | C₂₉H₄₄O₇ | **505.3194** | **505,3165** | Capitasterone (*ecdysteroids*) | Lei Fang et al. Molecules 2017, 22, 1310; |
| | C₃₃H₅₂O₉ | **593.3723** | **593,3689** | Nuatigenin-3-beta-D-gluco pyranoside or Agavoside A (*saponine monosaccharides*) | Gunstone, Frank D., John L. Harwood, and Albert J. Dijkstra (2007). The lipid CD-ROM. CRC Press |

**TABLE D**

| **Pomace** | **Formula** | **Exp m/z [M]⁺/[M+H]⁺** | **Calc m/z [M]⁺/[M+H]⁺** | **Example of a compound of natural origin** (class) | **references** |
|---|---|---|---|---|---|
| | C₂₀H₃₁O₃ | **320.238** | **320.235** | 20-hydroxyarachidonate (*fatty acid, triterpenoid*) | Ting Zheng et al. Front. Nutr. 2021, 8:715528 |
| Falanghina | C₁₇H₁₇O₁₀ | **381.082** | **381.0801** | 5-O-b-D-glucopyranosyl-8 hydroxypsoralen (*furanocoumarins*) | Boeira et al. Ciência Rural, v.52, n.9, 2022 |
| | C₂₂H₂₈O₁₀ | **453.1737** | **453.1760** | Floribundal (*Iridoids, cyclopentane pyran monoterpenes*) | Wang C, et al. Molecules. 2020 Jan 10;25(2):287. |
| | C₃₈H₄₈O₂ | **537.3621** | **537.3732** | C38-iso-Norcanthaxanthin (*keto-carotenoid*) | L. Ngamwonglumlert, Encyclopedia of Food Chemistry, 2019 |
| | C₃₇H₆₆O₉ | **655.4784** | **655.4785** | Salzmanolin *(Acetogenins, polyketide)* | Emerson F. Queiroz et al. J. Nat. Prod. 2003, 66, 755-758 |

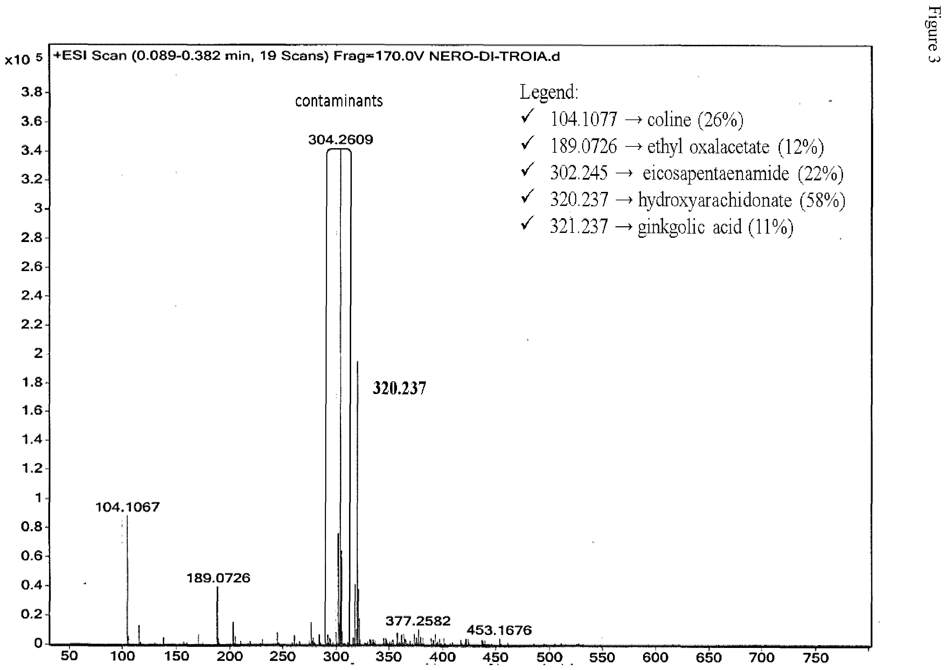

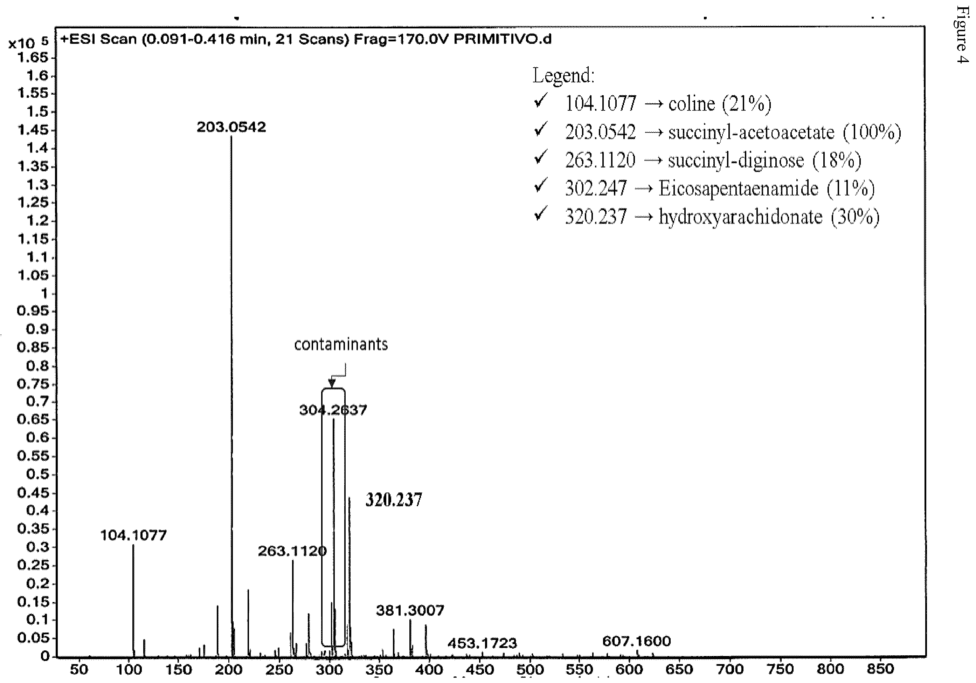

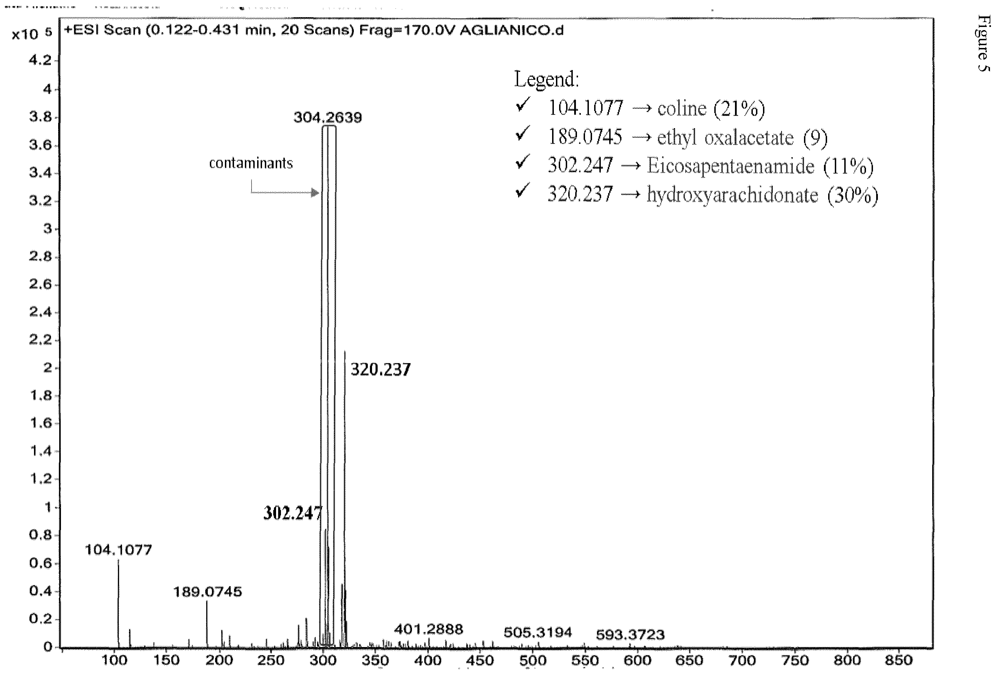

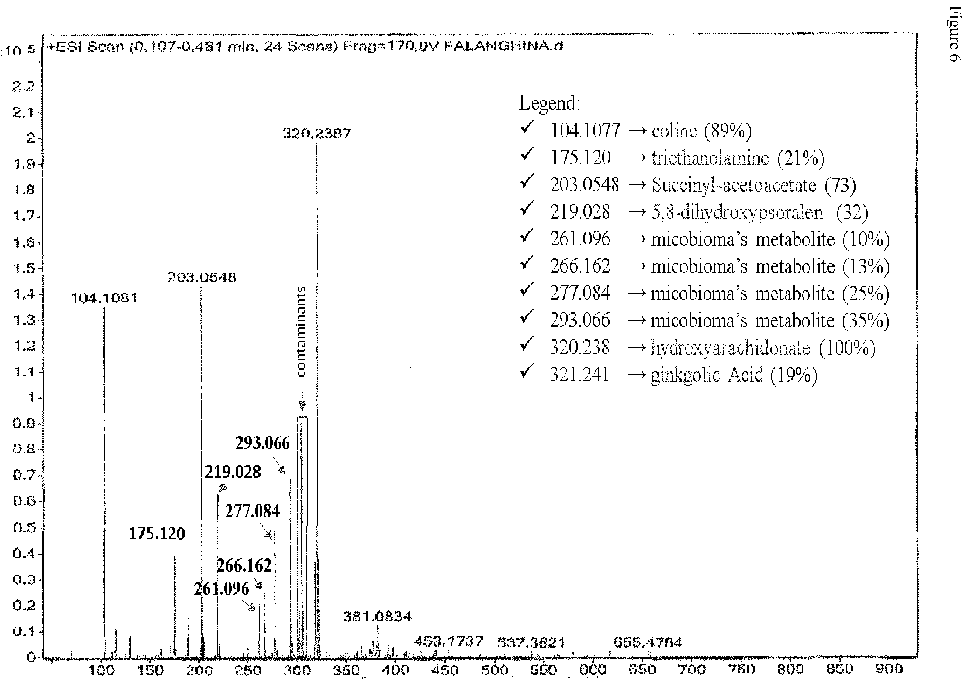

The results are also reported in the Figures 3-6, as images of the MS scans in positive ion mode of the samples. In most cases it was possible to associate a representative structure to the most intense m/z signals (see legend in each mass spectrum) by the exact monoisotopic mass conversion in elemental formula (ChemCalc), as thoughtful entry in available databases.

A preliminary assessment allowed the inventors to detect significant differences among the signal patterns of the three extracts from red vines and the one from white vine (Falanghina). The number and molecular diversity appear much more contained in the red samples with a prevalence of fatty acid derivatives compared to fermentation metabolites, as a clue of a greater stabilization of the red-derived pomaces.

### Conclusions

For some time now, the literature has given a plethora of papers, with numerous citations, aimed at defining and comparing the composition of pomaces by different grape varieties. Nevertheless, only a few works are aimed at the determination of lipids with the exclusion of some contributions inherent the antioxidant properties or as useful nutrients in dietary regimens (Ivana Dimic' et al., Antioxidants 2020, 9, 568 , Mariana Spinei et al., Foods 2021, 10(4), 867 and Yolanda Carmona-Jiménez et al., Molecules 2022, 27(20), 6980).

To the best of our knowledge, there are no studies that quantify substances such as eicosapentaenamide (EPM), 24-hydroxyarachidonate (24-HAC) and ginkgolic acid (GKA), yet present with dominant signals in the samples of red vines derived pomaces, especially in the Nero di Troia (NDT) sample

In particular, while EPM and 24-HAC, derivatives of polyunsaturated acids, and GKA, a 6-tridecylsalicylic acid, have aroused interest for their therapeutic potential (Sebastià Parets et al., Frontiers in Cell and Developmental Biology, 8 ,2020 and Cinzia Giordano et al., Int J Mol Sci 2020 Mar 26;21(7):2279), their possible use as surfactant-cleansing and emulsifying agent in cosmetics was not known.

Now, in light of our findings, we can affirm that pomace extracts, and in particular that of NdT, can be used as new and promising natural sources of polyunsaturated acid derivatives to modulate rheological properties in cosmetic production in a virtuous example of circular green economy.

## Claims

1. A gel composition for topical use comprising xanthan gum, a glyceric extract of the pomace of *Vitis vinifera* from the Nero di Troia vine and pharmaceutically acceptable excipients.

2. The composition according to claim 1, wherein the glyceric extract is present in amounts from 10% to 60% by weight, preferably from 20% to 50% by weight.

3. The composition according to claim 1, wherein the xanthan gum is present in an amount ranging from 0.5% to 4% by weight, preferably from 1% to 2% by weight.

4. The composition according to claim 1, comprising UVA, UVB sunscreens.

5. The composition according to claim 4, wherein the sunscreens are present in sufficient quantity to achieve a SPF50 protection.

6. The composition according to any of the preceding claims for use in a method for prevention of skin diseases.

7. The composition for the use according to claim 6, where the skin disease is a form of skin cancer, optionally melanoma.

8. The composition according to claim 3 for use in a method of preventing damage from oxidative stress due to sun exposure.

9. A glyceric extract of the pomace of *Vitis vinifera* from the Nero di Troia vine obtainable by ultrasonic extraction at room temperature for 4-10 minutes from a mixture of 50-80% by weight of glycerin and 20-50% of water.

10. A process for preparing a glyceric extract of the pomace of *Vitis vinifera* from the Nero di Troia vine by ultrasonic extraction at room temperature for 4-10 minutes from a mixture of 50-80% by weight of glycerin and 20-50% by weight of water.

11. A glyceric extract obtained by the process according to claim 9.

12. A process for the preparation of a composition according to any one of the claims from 1 to 4, comprising the mixture of xanthan gum, a glyceric extract of *Vitis vinifera* pomace from the Nero di Troia vine and pharmaceutically acceptable excipients.

## Patentansprüche

1. Gelzusammensetzung zur topischen Anwendung, umfassend Xanthan-gummi, einen glycerinhaltigen Extrakt aus dem Trester von *Vitis vinifera* der Rebsorte Nero di Troia und pharmazeutisch verträgliche Hilfsstoffe.

2. Zusammensetzung nach Anspruch 1, wobei der glycerinhaltige Extrakt in einer Menge von 10 % bis 60 % Gewichtsprozent, vorzugsweise von 20 % bis 50 % Gewichtsprozent, enthalten ist.

3. Zusammensetzung nach Anspruch 1, wobei das Xanthan-gummi in einer Menge von 0,5 % bis 4 % Gewichtsprozent, vorzugsweise von 1 % bis 2 % Gewichtsprozent, enthalten ist.

4. Zusammensetzung nach Anspruch 1, umfassend UVA- und UVB- Sonnenschutzfilter.

5. Zusammensetzung nach Anspruch 4, wobei die Sonnenschutzfilter in einer ausreichenden Menge vorhanden sind, um einen Lichtschutzfaktor (SPF) von 50 zu erreichen.

6. Zusammensetzung nach einem der vorhergehenden Ansprüche zur Verwendung in einem Verfahren zur Vorbeugung von Hauterkrankungen.

7. Zusammensetzung zur Verwendung nach Anspruch 6, wobei die Hauterkrankung eine Form von Hautkrebs ist, gegebenenfalls ein Melanom.

8. Zusammensetzung nach Anspruch 3 zur Verwendung in einem Verfahren zur Vorbeugung von durch Sonneneinstrahlung bedingten Schäden durch oxidativen Stress.

9. Glycerinhaltiger Extrakt aus dem Trester von *Vitis vinifera* der Rebsorte Nero di Troia, erhältlich durch Ultraschallextraktion bei Raumtemperatur über einen Zeitraum von 4 bis 10 Minuten aus einem Gemisch von 50-80 % Gewichtsprozent Glycerin und 20-50 % Wasser.

10. Verfahren zur Herstellung eines glycerinhaltigen Extrakts aus dem Trester von *Vitis vinifera* der Rebsorte Nero di Troia durch Ultraschallextraktion bei Raumtemperatur über einen Zeitraum von 4 bis 10 Minuten aus einem Gemisch, das 50 bis 80 % Gewichtsprozent Glycerin und 20 bis 50 % Gewichtsprozent Wasser umfasst.

11. Glycerinhaltiger Extrakt, erhältlich durch das Verfahren nach Anspruch 9.

12. Verfahren zur Herstellung einer Zusammensetzung nach einem der Ansprüche 1 bis 4, umfassend das Gemisch von Xanthan-gummi, einem glycerinhaltigen Extrakt aus dem Trester von *Vitis vinifera* der Rebsorte Nero di Troia und pharmazeutisch verträglichen Hilfsstoffen.

## Revendications

1. Une composition gélifiée à usage topique comprenant de la gomme xanthane, un extrait glycériné du marc de *Vitis vinifera* issu du cépage Nero di Troia, et des excipients pharmaceutiquement acceptables.

2. La composition selon la revendication 1, dans laquelle l'extrait glycériné est présent en une quantité comprise entre 10 % et 60 % en poids, de préférence entre 20 % et 50 % en poids.

3. La composition selon la revendication 1, dans laquelle la gomme xanthane est présente en une quantité comprise entre 0,5 % et 4 % en poids, de préférence entre 1 % et 2 % en poids.

4. La composition selon la revendication 1, comprenant des filtres solaires UVA et UVB.

5. La composition selon la revendication 4, dans laquelle les filtres solaires sont présents en quantité suffisante pour atteindre une protection SPF50.

6. La composition selon l'une quelconque des revendications précédentes pour une utilisation dans une méthode de prévention des maladies cutanées.

7. La composition pour l'utilisation selon la revendication 6, dans laquelle la maladie cutanée est une forme de cancer de la peau, optionnellement un mélanome.

8. La composition selon la revendication 3 pour une utilisation dans une méthode de prévention des dommages dus au stress oxydatif provoqué par l'exposition solaire.

9. Un extrait glycériné du marc de *Vitis vinifera* issu du cépage Nero di Troia, obtenable par extraction ultrasonique à température ambiante pendant 4 à 10 minutes à partir d'un mélange comprenant de 50 à 80 % en poids de glycérine et de 20 à 50 % d'eau.

10. Un procédé de préparation d'un extrait glycériné du marc de *Vitis vinifera* issu du cépage Nero di Troia, par extraction ultrasonique à température ambiante pendant 4 à 10 minutes à partir d'un mélange comprenant de 50 à 80 % en poids de glycérine et de 20 à 50 % en poids d'eau.

11. Un extrait glycériné obtenu par le procédé selon la revendication 9.

12. Un procédé de préparation d'une composition selon l'une quelconque des revendications 1 à 4, comprenant le mélange de gomme xanthane, d'un extrait glycériné du marc de *Vitis vinifera* issu du cépage Nero di Troia et d'excipients pharmaceutiquement acceptables.
